# EUROPÄISCHE PATENTSCHRIFT

(11) **EP 2 247 329 B1**
(45) Veröffentlichungstag und Bekanntmachung des Hinweises auf die Patenterteilung: **16.05.2018**
(21) Anmeldenummer: 09714163.4
(22) Anmeldetag: 27.02.2009
(51) Int. Cl.: A61M 5/44, A61M 1/28, A61M 1/16

(54) **VERFAHREN UND VORRICHTUNG ZUM ERWÄRMEN VON LÖSUNGEN, VORZUGSWEISE DIALYSELÖSUNGEN**
METHOD AND DEVICE FOR HEATING SOLUTIONS, PREFERABLY DIALYSIS SOLUTIONS
PROCÉDÉ ET DISPOSITIF POUR RÉCHAUFFER DES SOLUTIONS, DE PRÉFÉRENCE DES SOLUTIONS DE DIALYSE

(30) Priorität: 29.02.2008 DE 102008011828
(43) Veröffentlichungstag der Anmeldung: 10.11.2010
(73) Patentinhaber: Fresenius Medical Care Deutschland GmbH, 61352 Bad Homburg (DE)
(72) Erfinder: HEDMANN, Frank L., 97332 Volkach (DE); KLATTE, Stephan, 31582 Nienburg (Weser) (DE)
(74) Vertreter: Laufhütte, Dieter
(86) Internationale Anmeldenummer: PCT/EP2009/001435
(87) Internationale Veröffentlichungsnummer: WO 2009/106354

(56) Entgegenhaltungen:
- DE-A1- 19 752 578
- US-A- 3 863 048
- US-A- 3 908 111
- US-A1- 2004 170 409
- US-A1- 2004 265 168
- US-A1- 2009 012 456

## Beschreibung

Die Erfindung betrifft ein Verfahren zum Erwärmen von Lösungen, vorzugsweise Dialyselösungen, nach dem Oberbegriff des Anspruchs 1.

Die vorzugsweise mit dem Verfahren gemäß der Erfindung behandelten Dialyselösungen werden beispielsweise auf dem Gebiet der Peritonealdialyse eingesetzt. Bei der Peritonealdialyse soll die in die Bauchhöhle zu infundierende Dialyselösung ungefähr auf Körpertemperatur temperiert sein. Dies wird zum einen vom Patienten als angenehm empfunden und ist zum anderen der Gesundheit zuträglich. Eine andere Anwendung liegt auf dem Gebiet der Infusionstechnik, der Bluttransfusionstechnik oder anderen ähnlichen Gebieten, in welchen die Flüssigkeiten erwärmt werden müssen.

Ebenso betrifft die Erfindung eine Vorrichtung, insbesondere in einem Peritonealdialysegerät, zum Erwärmen von Lösungen, vorzugsweise Dialyselösungen, nach dem Oberbegriff des Anspruchs 10.

Es ist bereits bekannt, bei der Peritonealdialyse einzusetzende Dialyselösungen vor der Infusion zu temperieren. So beschreibt beispielsweise die EP 0 956 876 B1 eine Kassette zur Förderung von Dialyseflüssigkeiten, bei der die Dialyseflüssigkeit vor der Infusion aufgeheizt wird. Hierzu sind die die Dialyseflüssigkeit führenden Leitungen in einem beheizbaren Bereich der Kassette spiralförmig angeordnet.

Gemäß der WO 88/09186 A1 und der WO 97/09074 A2 wird die aufzuwärmende Dialyselösung in einem Beutel vorgelegt und in dieser Form erwärmt. Dabei dient in der WO 97/09074 A2 eine Heizplatte zur Erwärmung des Beutels.

Bei der Erwärmung der Dialyselösung soll die Aufwärmzeit möglichst minimiert werden. Allerdings kann die Anfangstemperatur der Heizplatte zum Aufheizen der Raumtemperatur aufweisenden Dialyselösung nicht beliebig hoch gewählt werden. Im Fall der Verwendung einer Heizplatte muss hier einerseits auf die Hitzestabilität der Dialyselösung selbst und andererseits auf die Hitzestabilität des Beutelmaterials Rücksicht genommen werden. Darüber hinaus muss gerade für den Fall, dass das Heizplattensystem für den Bediener zugänglich ist, dieses so ausgestaltet sein, dass eine Verletzungsgefahr durch Verbrennungen an einer entsprechend heißen Heizplatte sicher ausgeschlossen werden kann.

Ein weiteres Problem besteht in dem sogenannten Überschwingen der Aufheiztemperatur, das sich wie folgt ergibt. Um ein möglichst schnelles Erwärmen des Beutelinhalts zu erreichen, könnte die Heizvorrichtung vorzugsweise bei einer maximalen Grenztemperatur betrieben werden, die den zuvor wiedergegebenen Randbedingungen Rechnung trägt. Wird jedoch während dem Aufheizen der Heizplatte oder nach entsprechendem Erreichen der gewünschten Solltemperatur der Beutel von der Heizplatte entfernt, so entsteht auf der Heizplatte Stauwärme und dadurch ein Hitzestau, der die Temperatur kurzfristig über die erlaubte Grenztemperatur ansteigen läßt. Dieses Überschreiten der Grenztemperatur kann als Überschwingen bezeichnet werden. Bei diesem unkontrollierten Überschreiten der Grenztemperatur besteht akute Verbrennungsgefahr für den Benutzer.

Ein gattungsgemäßes Verfahren zum Erwärmen von Lösungen ist bereits aus der DE 197 52 578 A1 bekannt.

Die Aufgabe der vorliegenden Erfindung ist es daher, ein gattungsgemäßes Verfahren und eine Vorrichtung zum Erwärmen von Lösungen derart weiterzubilden, dass ein die Lösung enthaltender Beutel möglichst schnell auf die gewünschte Endtemperatur aufgeheizt werden kann, ohne dass die Gefahr von Temperaturüberschwingern beim Aufheizen der Heizplatte besteht.

Erfindungsgemäß wird diese Aufgabe durch das Verfahren gemäß der Merkmalskombination nach Anspruch 1 gelöst. Demnach ist ein Verfahren zum Erwärmen von Lösungen, vorzugsweise Dialyselösungen, auf eine gewünschte Solltemperatur vorgeschlagen, bei dem eine in einem Beutel vorgelegte Lösung auf einem eine Heizplatte umfassenden Heizplattensystem mittels einer Zweipunktregelung aufgeheizt wird. Nach der vorliegenden Erfindung variieren die die untere Einschaltschwelle der Heizplatte bildende Temperatur und die die obere Abschaltschwelle der Heizplatte bildende Temperatur. Hierdurch kann das unerwünschte Überschwingen der Temperatur der Heizplatte beim Aufheizvorgang sicher verhindert werden.

Bevorzugte Ausgestaltungen der Erfindung ergeben sich aus den sich an den Hauptanspruch anschließenden Unteransprüchen.

Gemäß einer ersten vorteilhaften Ausgestaltung der Erfindung wird die Temperatur T₁ des die Lösung enthaltenden Beutels mittels eines ersten Temperatursensors gemessen, während die Temperatur T₂ eines im Heizplattensystem angeordneten Heizelementes über einen zweiten Temperatursensor gemessen wird.

Vorteilhaft wird als die die obere Abschaltschwelle bildende Temperatur T₂ eine vorher festgelegte Vorsteuertemperatur Tᵥ eingesetzt. Diese Vorsteuertemperatur Tᵥ wird für diskrete Beuteltemperaturen T₁ vorab bestimmt.

Besonders vorteilhaft wird das erfindungsgemäße Verfahre mittels einer Zweipunktregelung durchgeführt, bei der iterativ folgende Schritte ablaufen:
- Messung der Temperatur T₁ des die Lösung enthaltenden Beutels;
- Setzen der Vorsteuertemperatur Tᵥ ;
- Heizen des Heizelementes bis die Vorsteuertemperatur Tᵥ erreicht ist.

Dieses Verfahren wird so lange wiederholt bis der Beutel die gewünschte Temperatur aufweist, wobei nach dem Aufheizen des Heizelements bis zum Erreichen der Vorsteuertemperatur Tᵥ das Heizelement abkühlt bis es die zwischenzeitlich erreichte Temperatur des Lösungsbeutels T₁ erreicht hat. Anschließend wird es in den nachfolgenden Iterationsschritten jeweils wieder bis zum Erreichen der Vorsteuertemperatur Tᵥ aufgeheizt.

Vorzugsweise wird die Vorsteuertemperatur so gewählt, dass ein Überschwingen der Temperatur über die maximale Heizplattentemperatur Tₘₐₓ nicht möglich ist.

Gemäß einer besonders vorteilhaften Ausgestaltung der Erfindung wird der Einschaltpunkt zum Einschalten des Heizelementes dadurch bestimmt, dass ein vorbestimmtes Verhältnis von der die obere Abschaltschwelle bildenden Temperatur T₂ zu der Temperatur T₁ des die Lösung enthaltenden Beutels erreicht wird.

Wiederum von besonderem Vorteil ist es, wenn die Temperatur T₁ des die Lösung enthaltenden Beutels bestimmt wird, wenn der Einfluß des Heizelementes auf den die Temperatur T₁ des die Lösung enthaltenden Beutels messenden Sensor einen Grenzwert unterschritten hat. Hierdurch läßt sich folgendes Phänomen berücksichtigen. Da aufgrund des Heizplattenaufbaus der Temperatursensor zur Messung der Temperatur T₁ nicht völlig thermisch entkoppelt ist, kann die gemessene Temperatur an diesem Sensor nicht direkt der Lösungstemperatur zugeordnet werden. Nach Abschluß des Heizvorganges nimmt der Sensor zur Messung der Temperatur T₂ die Temperatur des Heizelementes an, der Sensor zur Messung der Temperatur T₁ nimmt eine Temperatur ein, die zwischen der tatsächlichen Lösungstemperatur und der des Heizelementes liegt. Entsprechend der vorgenannten vorteilhaften Ausgestaltung des erfindungsgemäßen Verfahrens wird nun gewartet bis der Einfluß des Heizelementes auf den die Temperatur T₁ des die Lösungstemperatur messenden Sensors einen vorbestimmten Grenzwert unterschritten hat. Hierdurch wird sichergestellt, dass die entstandene Wärmeenergie abgebaut ist und in die Lösung übergegangen ist, damit der Einfluß des Heizelementes auf den die Temperatur T₁ messenden Sensor minimiert ist und sich nun die Lösungstemperatur mit größerer Genauigkeit bestimmen läßt.

Der vorgenannte Grenzwert läßt sich vorteilhaft dadurch bestimmen, dass die Temperatur T₁ des die Lösung enthaltenden Beutels aus dem Gradienten der die Temperaturen messenden Sensoren bestimmt wird. Hier kann vorteilhaft der Gradient der Temperaturänderungen T₁ und T₂, der Gradient der Temperaturänderung T₁ zu T₂ oder die Temperaturdifferenz von T₁ und T₂ bestimmt werden.

Die vorgenannte Aufgabe wird auch durch eine Vorrichtung gemäß Anspruch 10 gelöst. Bevorzugte Ausgestaltungen dieser Vorrichtung ergeben sich aus den sich an den Anspruch 10 anschließenden Unteransprüchen.

Das vorgenannte Verfahren und/oder die vorgenannte Vorrichtung können in einem Gerät für die Peritonealdialyse gemäß der US 2006/0195064 A1 und der US 2007/0112297 A1 eingesetzt werden. Auf den Inhalt der US 2006/0195064 A1 und der US 2007/0112297 A1 wird Bezug genommen und die gegenständliche Beschreibung dieser Schriften wird durch Bezugnahme zum Gegenstand der vorliegenden Anmeldung gemacht.

Weitere Merkmale, Einzelheiten und Vorteile der Erfindung werden anhand eines in der Zeichnung dargestellten Ausführungsbeispiels im folgenden näher erläutert. Es zeigen:
- Figur 1:: eine schematische Darstellung eines Heizplattensystems zur Durchführung des erfindungsgemäßen Verfahrens und
- Figur 2:: den Temperaturverlauf eines typischen Aufheizvorgangs aufgetragen über die Zeit gemäß dem erfindungsgemäßen Verfahren.

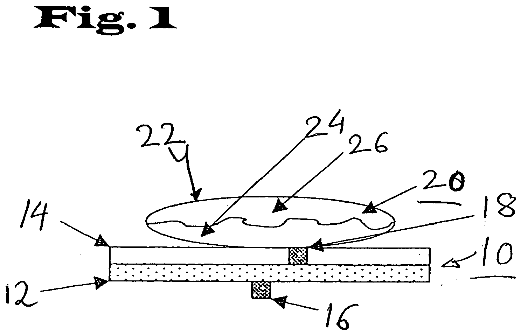

In Figur 1 ist ein Heizplattensystem 10 in seinem schematischen Aufbau gezeigt, wie es zum Aufheizen von Dialyselösungen, die in der Peritonealdialyse eingesetzt werden, verwendet wird. Das Heizplattensystem 10 besteht aus einem Heizelement 12 und einer auf dieser aufgebrachten Heizplatte 14, die beispielsweise aus Aluminium besteht. Dem Heizelement 12 ist ein Sensor 16 zugeordnet, der die Temperatur T₂ des Heizelementes mißt. Demgegenüber ist in der Heizplatte 14 ein Sensor 18 eingebettet, der mit einem die zu erhitzende Dialyselösung 20 enthaltenden Beutel 22 in Berührung steht, wie dies in Figur 1 schematisch dargestellt ist. Während des Aufheizens der in dem Beutel 22 vorgelegten Dialyselösung 20 mittels der Heizplatte 14 bildet sich eine warme Lösungszone 24 in unmittelbarer Nachbarschaft der aufgeheizten Heizplatte und eine kalte Lösungszone 26 auf der von der Heizplatte 14 abgekehrten Seite des Beutels 22.

Die Dialyselösung 20 im Beutel 22 soll möglichst kurzfristig auf 37°C erwärmt werden. Um möglichst kurze Aufrüstzeiten für die Peritonealdialyse, in der die Dialyseflüssigkeit verwendet werden soll, zu ermöglichen, soll dieser Zeitraum beispielsweise 45 Minuten umfassen. Nun ist zu berücksichtigen, dass sich das Füllvolumen 22 während der Behandlung ändert, da dieser Beutel für die Einlaufphase bei der Peritonealdialyse verwendet wird.

Die möglichst kurze Zeit zum Aufwärmen auf 37°C muss mittels einer maximalen Heizplattentemperatur Tₘₐₓ von 55°C erreicht werden. Diese maximale Heizplattentemperatur ist dadurch vorgegeben, dass eine höhere Temperatur zu einer möglichen Verletzung des Patienten führen könnte, wenn er die Heizplatte während des Betriebs berührt.

Aufgrund der vorgenannten Eigenschaften ist in der Regel das genaue Volumen des Dialyselösungsbeutels 20 nicht bekannt. Auch die Starttemperatur der Dialyselösung im Lösungsbeutel ist in der Regel nicht bekannt. Es muss auch berücksichtigt werden, dass der Temperatursensor 18 des Beutels durch die Temperatur des Heizplattenträgers oder Heizelementes beeinflußt werden kann.

Die vorgenannten Anforderungen können mittels der erfindungsgemäßen Verfahrensführung dadurch erreicht werden, dass immer nur so viel Wärmeenergie in den Heizplattenträger eingespeist wird, dass ein Überschwingen über die maximale Temperatur Tₘₐₓ sicher verhindert wird. Der Aufheizvorgang der Heizplatte 14 über das Heizelement 12 des Heizplattensystems 10 erfolgt mittels eines hier nicht näher dargestellten im Stand der Technik aber weithin bekannten Zweipunktreglers. Erfindungsgemäß werden gegenüber den bekannten Zweipunktreglern nach dem Stand der Technik aber die obere Abschaltschwelle und auch die untere Einschaltschwelle über die Zeit variiert. Dabei wird die obere Abschaltschwelle durch eine vorher festgelegte Vorsteuertemperatur Tᵥ begrenzt.

Der Einschaltpunkt dagegen kann ermittelt werden, indem die Temperatur, die mit dem Sensor 18 gemessen wird, nämlich die Temperatur T₁ im richtigen Verhältnis mit der Temperatur des Heizelements 12, die mit dem Sensor 16 gemessen wird, nämlich der Temperatur T₂, steht.

Die Vorsteuertemperatur wird gemäß dem hier dargestellten Ausführungsbeispiel wie folgt bestimmt. Um die Energiemenge des Heizelementes so zu dosieren, dass kein Überschwingen der Temperatur über die Maximaltemperatur Tₘₐₓ entsteht, wird die Vorsteuertemperatur Tᵥ in Abhängigkeit zur Heizplattentemperatur ermittelt. Ziel ist es hierbei, eine Vorsteuertemperatur zu wählen, die es erlaubt, einen kalten, beispielsweise 5 l enthaltenden Beutel 20 mit Dialyselösung 20 von einer noch aufheizenden Heizplatte 14 abzunehmen, ohne dass ein Überschwingen über die maximale Temperatur Tₘₐₓ erfolgt.

Dabei fällt es dem Heizelement sehr leicht, aufgrund des großen Temperaturgefälles zwischen dem Heizelement 14 und dem Beutel 22 Wärmeenergie in die Lösung 20 abzugeben. In dem Moment, in welchem der Beutel von der Heizplatte 14 entfernt wird, entsteht hier aber eine Stauwärme, die nicht mehr so schnell in die Umgebung abgeführt werden kann. Dies führt zu dem zuvor beschriebenen Überhitzen des Heizplattensystems 10.

Um dieses vorbeschriebene Erhitzen zu verhindern, muß die Vorsteuertemperatur Tᵥ einerseits relativ niedrig gewählt werden, jedoch so hoch, um ein schnelles Erwärmen der Lösung sicherzustellen.

Dabei wird erfindungsgemäß die gemessene Lösungsbeuteltemperatur T₁ in ein festes Verhältnis zu der Vorsteuertemperatur Tᵥ gesetzt. Dieser Zusammenhang wird anhand des in der folgenden Tabelle wiedergegebenen Ausführungsbeispiels dargestellt:

| T₁ | T_{V} |
|---|---|
| 5°C | 35°C |
| 10°C | 37°C |
| 15°C | 39°C |
| 20°C | 41°C |
| 25°C | 43°C |
| 30°C | 45°C |
| 35°C | 47°C |

Die Zuordnung der Werte in der vorgenannten Tabelle bedeuten, dass beispielsweise bei der Messung eines Temperaturwertes T₁ von 15°C der vorbestimmte Vorsteuertemperaturwert Tᵥ auf 39°C als obere Abschaltschwelle gesetzt wird.

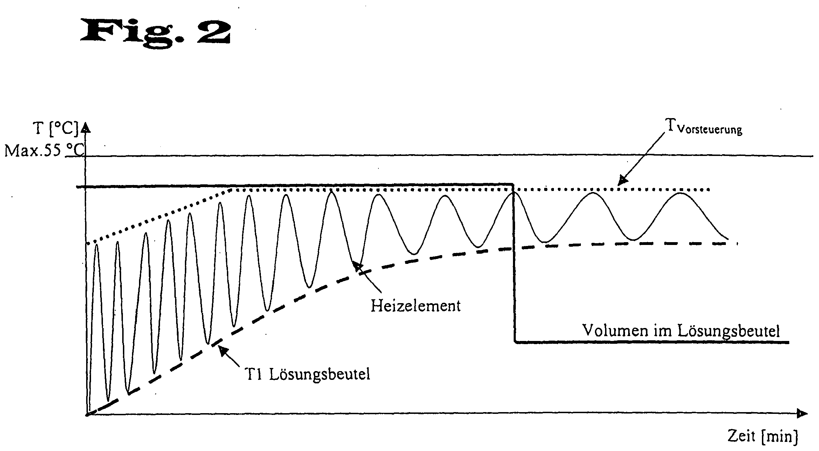

Anhand des in Figur 2 dargestellten Temperaturverlaufs läßt sich nun der Ablauf des erfindungsgemäßen Verfahrens erläutern. Hier wird iterativ wie folgt vorgegangen:

In einem ersten Schritt wird die Lösungsmitteltemperatur T₁ durch den Sensor 18 bestimmt. In Abhängigkeit von dieser gemessenen Temperatur T₁ wird die Vorsteuertemperatur Tᵥ aus der vorgenannten Tabelle ausgewählt. Es wird nun das Heizplattensystem 10 aufgeheizt, bis das Heizelement 12 die Vorsteuertemperatur Tᵥ erreicht hat. Dies wird über den Sensor 16 und den Temperaturverlauf T₂ des Heizelements 12 überwacht.

Im nächsten Schritt wird die Lösungstemperatur des erwärmten Teils der Lösung 20 gemessen und der vorgenannte Verfahrensablauf wiederholt, bis die gewünschte Lösungsmitteltemperatur erreicht wird.

Bei dem erfindungsgemäßen Verfahren ist es nicht notwendig, dass der Wärmeübergangswiderstand zwischen dem Heizelement 14 und dem Beutel 22 mit der enthaltenden Lösung 20 bekannt ist. Es können hier verschiedene Materialien verwendet werden, beispielsweise Aluminium für den Heizplattenträger und beispielsweise PVC für den Lösungsmittelbeutel 22.

Die Auflagefläche des Beutels auf dem Heizplattenträger kann je nach Beutelgröße und Beutelvolumen variieren und muß dem System auch nicht bekannt sein, um das erfindungsgemäße Verfahren durchzuführen.

Gemäß dem erfindungsgemäßen Verfahren kann auch die Totzeit, in welcher die erzeugte Wärmeenergie aus dem Heizelement 12 in den Lösungsbeutel 22 übergegangen ist und dort die Lösung erwärmt hat, berücksichtigt werden.

## Patentansprüche

1. Verfahren zum Erwärmen von Lösungen, vorzugsweise Dialyselösungen, auf eine gewünschte Solltemperatur, bei dem eine in einem Beutel (22) vorgelegte Lösung auf einem eine Heizplatte (12) umfassenden Heizplattensystem (10) mittels einer Zweipunktregelung aufgeheizt wird,
**dadurch gekennzeichnet,**
**dass** die die untere Einschaltschwelle der Heizplatte (12) bildende Temperatur und die die obere Abschaltschwelle der Heizplatte (12) bildende Temperatur iterativ über die Zeit variieren.

2. Verfahren nach Anspruch 1, **dadurch gekennzeichnet, dass** die Temperatur T₁ des die Lösung enthaltenden Beutels (22) mittels eines ersten Temperatursensors (18) gemessen wird, während die Temperatur T₂ eines im Heizplattensystem angeordneten Heizelementes über einen zweiten Temperatursensor (16) gemessen wird.

3. Verfahren nach Anspruch 1 oder 2, **dadurch gekennzeichnet, dass** als die die obere Abschaltschwelle bildende Temperatur T₂ eine vorher festgelegte Vorsteuertemperatur Tᵥ eingesetzt wird.

4. Verfahren nach einem der Ansprüche 1 bis 3, **dadurch gekennzeichnet, dass** die Vorsteuertemperatur für diskrete Beuteltemperaturen T₁ vorab bestimmt wird.

5. Verfahren nach einem der vorangehenden Ansprüche, **dadurch gekennzeichnet, dass** bei der Zweipunktregelung iterativ folgende Schritte ablaufen:
- Messung der Temperatur T₁ des die Lösung enthaltenden Beutels (22);
- Setzen der Vosteuertemperatur Tᵥ ;
- Heizen des Heizelementes bis die Vorsteuertemperatur Tᵥ erreicht ist.

6. Verfahren nach einem der vorangehenden Ansprüche, **dadurch gekennzeichnet, dass** die Vorsteuertemperatur so gewählt ist, dass ein Überschwingen der Temperatur über die maximale Heizplattentemperatur Tₘₐₓ nicht möglich ist.

7. Verfahren nach einem der vorangehenden Ansprüche, **dadurch gekennzeichnet, dass** der Einschaltpunkt zum Einschalten des Heizelementes (12) dadurch bestimmt wird, dass ein vorbestimmtes Verhältnis von der die obere Abschaltschwelle bildenden Temperatur T₂ zu der Temperatur T₁ des die Lösung enthaltenen Beutels (22) erreicht wird.

8. Verfahren nach einem der vorangehenden Ansprüche, **dadurch gekennzeichnet, dass** die Temperatur T₁ des die Lösung enthaltenden Beutels (22) bestimmt wird, wenn der Einfluß des Heizelementes (12) auf den die Temperatur T₁ des die Lösung enthaltenden Beutels (22) messenden Sensor einen Grenzwert unterschritten hat.

9. Verfahren nach Anspruch 8, **dadurch gekennzeichnet, dass** die Temperatur T₁ des die Lösung enthaltenen Beutels (22) aus dem Gradienten der die Temperaturen messenden Sensoren (16, 18) bestimmt wird.

10. Vorrichtung zum Erwärmen von Lösungen, vorzugsweise Dialyselösungen, auf eine gewünschte Solltemperatur bei dem eine in einem Beutel (22) vorgelegte Lösung auf einem eine Heizplatte (12) umfassenden Heizplattensystem mittels (10) einer Zweipunktregelung aufheizbar ist,
**dadurch gekennzeichnet,**
**dass** das Heizplattensystem (10) Mittel aufweist, mittels der die untere Einschaltschwelle der Heizplatte (12) bildende Temperatur und die die obere Ausschaltschwelle der Heizplatte (12) bildende Temperatur iterativ über die Zeit variierbar sind.

11. Vorrichtung nach Anspruch 10, **dadurch gekennzeichnet, dass** die Mittel Speicher zum Abspeichern diskreter Temperaturwerte aufweisen.

12. Vorrichtung nach Anspruch 10 oder 11, **dadurch gekennzeichnet, dass** ein erster Temperatursensor (16) im Heizelement (12) des Heizplattensystems (10) eingebettet ist.

13. Vorrichtung nach einem der Ansprüche 10 bis 12, **dadurch gekennzeichnet, dass** ein Temperatursensor (18) auf dem Heizelement (12) des Heizplattensystems (10) angeordnet ist.

## Claims

1. A method for the heating of solutions, preferably dialysis solutions, to a wanted desired temperature, wherein a solution presented in a bag (22) is heated on a heater system (10) including a heater (12) by means of a two-step control,
**characterized in that**
the temperature forming the lower switch-on threshold of the heater (12) and the temperature forming the upper switch-off threshold of the heater (12) vary iteratively over time.

2. A method in accordance with claim 1, **characterized in that** the temperature T₁ of the bag (22) containing the solution is measured by means of a first temperature sensor (18), whereas the temperature T₂ of a heating element arranged in the heater system is measured via a second temperature sensor (16).

3. A method in accordance with either of claims 1 or 2, **characterized in that** a previously fixed precontrol temperature Tᵥ is used as the temperature T₂ forming the upper switch-off threshold.

4. A method in accordance with one of the claims 1 to 3, **characterized in that** the precontrol temperature for discrete bag temperatures T₁ is determined in advance.

5. A method in accordance with one of the preceding claims, **characterized in that** the following steps take place iteratively in the two-point control:
- measuring the temperature T ₁ of the bag (22) containing the solution;
- setting the precontrol temperature Tᵥ;
- heating the heating element until the precontrol temperature Tᵥ is reached.

6. A method in accordance with one of the preceding claims, **characterized in that** the precontrol temperature is selected such that an overshooting of the temperature beyond the maximum heater temperature Tₘₐₓ is not possible.

7. A method in accordance with one of the preceding claims, **characterized in that** the switch-on point for the switching on of the heating element (12) is determined **in that** a predetermined ratio of the temperature T₂ forming the upper switch-off threshold to the temperature T₁ of the bag (22) containing the solution is reached.

8. A method in accordance with one of the preceding claims, **characterized in that** the temperature T₁ of the bag (22) containing the solution is determined when the influence of the heating element (12) on the sensor measuring the temperature T₁ of the bag (22) containing the solution has fallen below a limit value.

9. A method in accordance with claim 8, **characterized in that** the temperature T₁ of the bag (22) containing the solution is determined from the gradient of the sensors (16, 18) measuring the temperatures.

10. An apparatus for the heating of solutions, preferably dialysis solutions, to a wanted desired temperature, wherein a solution presented in a bag (22) is heated on a heater system (10) including a heater (12) by means of a two-step control,
**characterized in that**
the heater system (10) has means by means of which the temperature forming the lower switch-on threshold of the heater (12) and the temperature forming the upper switch-off threshold of the heater (12) are iteratively variable over time.

11. An apparatus in accordance with claim 10, **characterized in that** the means have stores for storing discrete temperature values.

12. An apparatus in accordance with either of claims 10 or 11, **characterized in that** a first temperature sensor (16) is embedded in the heating element (12) of the heater system (10).

13. An apparatus in accordance with any one of the claims 10 to 12, **characterized in that** a temperature sensor (18) is arranged on the heating element (12) of the heater system (10).

## Revendications

1. Procédé pour réchauffer des solutions, de préférence des solutions de dialyse, à une température de consigne souhaitée, dans lequel une solution présentée dans une poche (22) est chauffée sur un système de plaque chauffante (10) comprenant une plaque chauffante (12) au moyen d'une régulation à deux positions,
**caractérisé en ce que**
la température représentant le seuil inférieur de mise sous tension de la plaque chauffante (12) et la température représentant le seuil supérieur d'arrêt de la plaque chauffante (12) varient dans le temps de manière itérative.

2. Procédé selon la revendication 1, **caractérisé en ce que** la température T₁ de la poche (22) contenant la solution est mesurée au moyen d'un premier capteur de température (18), tandis que la température T₂ d'un élément chauffant disposé dans le système de plaque chauffante est mesurée par un second capteur de température (16).

3. Procédé selon la revendication 1 ou 2, **caractérisé en ce qu'**une température pilote Tᵥ définie auparavant est utilisée comme la température T₂ représentant le seuil supérieur d'arrêt.

4. Procédé selon l'une des revendications 1 à 3, **caractérisé en ce que** la température pilote est déterminée au préalable pour des températures de poche discrètes T₁.

5. Procédé selon l'une des revendications précédentes, **caractérisé en ce que**, lors de la régulation à deux positions, les étapes suivantes se déroulent de manière itérative :
- mesure de la température T₁ de la poche (22) contenant la solution ;
- réglage de la température pilote Tᵥ ;
- chauffage de l'élément chauffant jusqu'à ce que la température pilote Tᵥ soit atteinte.

6. Procédé selon l'une des revendications précédentes, **caractérisé en ce que** la température pilote est choisie de telle manière qu'un dépassement de la température au-dessus de la température maximale de plaque chauffante Tₘₐₓ est impossible.

7. Procédé selon l'une des revendications précédentes, **caractérisé en ce que** le point de mise sous tension pour la mise sous tension de l'élément chauffant (12) est déterminé par le fait qu'un rapport prédéfini de la température T₂ représentant le seuil supérieur d'arrêt à la température T₁ de la poche (22) contenant la solution est atteint.

8. Procédé selon l'une des revendications précédentes, **caractérisé en ce que** la température T₁ de la poche (22) contenant la solution est déterminée quand l'influence de l'élément chauffant (12) sur le capteur mesurant la température T₁ de la poche (22) contenant la solution est inférieure à une valeur limite.

9. Procédé selon la revendication 8, **caractérisé en ce que** la température T₁ de la poche (22) contenant la solution est déterminée à partir des gradients des capteurs (16, 18) mesurant les températures.

10. Dispositif pour réchauffer des solutions, de préférence des solutions de dialyse, à une température de consigne souhaitée, dans lequel une solution présentée dans une poche (22) peut être chauffée sur un système de plaque chauffante (10) comprenant une plaque chauffante (12) au moyen d'une régulation à deux positions,
**caractérisé en ce que**
le système de plaque chauffante (10) comporte des moyens, au moyen desquels la température représentant le seuil inférieur de mise sous tension de la plaque chauffante (12) et la température représentant le seuil supérieur d'arrêt de la plaque chauffante (12) peuvent être variées dans le temps de manière itérative.

11. Dispositif selon la revendication 10, **caractérisé en ce que** les moyens comportent des mémoires pour enregistrer des valeurs discrètes de température.

12. Dispositif selon la revendication 10 ou 11, **caractérisé en ce qu'**un premier capteur de température (16) est enrobé dans l'élément chauffant (12) du système de plaque chauffante (10).

13. Dispositif selon l'une des revendications 10 à 12, **caractérisé en ce qu'**un capteur de température (18) est disposé sur l'élément chauffant (12) du système de plaque chauffante (10).
